# EUROPÄISCHE PATENTSCHRIFT

(11) **EP 1 501 788 B1**
(45) Veröffentlichungstag und Bekanntmachung des Hinweises auf die Patenterteilung: **14.04.2010**
(21) Anmeldenummer: 03720529.1
(22) Anmeldetag: 25.04.2003
(51) Int. Cl.: C07C 253/30, C07C 255/56

(54) **VERFAHREN ZUR HERSTELLUNG VON 3-TRIFLUORMETHYLPHENYL-4-CYANOBENZYLKETON**
METHOD FOR PRODUCING 3-TRIFLUOROMETHYLPHENYL-4-CYANOBENZYL KETONE
PROCEDE POUR PRODUIRE DE LA CETONE 3-TRIFLUOROMETHYLE PHENYLE-4-CYANOBENZYLE

(30) Priorität: 26.04.2002 DE 10218764
(43) Veröffentlichungstag der Anmeldung: 02.02.2005
(73) Patentinhaber: BASF SE, 67056 Ludwigshafen (DE)
(72) Erfinder: ENGEL, Stefan, 55268 Nieder-Olm (DE); KEIL, Michael, 67251 Freinsheim (DE); OTT, Christian, 67346 Speyer (DE); RACK, Michael, 69123 Heidelberg (DE)
(86) Internationale Anmeldenummer: PCT/EP2003/004327
(87) Internationale Veröffentlichungsnummer: WO 2003/091203

(56) Entgegenhaltungen:
- DATABASE WPI Section Ch, Week 199245 Derwent Publications Ltd., London, GB; Class B05, AN 1992-369413 XP002250631 & JP 04 270248 A (NIHON NOYAKU CO LTD), 25. September 1992 (1992-09-25) in der Anmeldung erwähnt

## Beschreibung

Die vorliegende Erfindung betrifft ein Verfahren zur Herstellung von 3-Trifluormethylphenyl-4-cyanobenzylketon.

α-Phenylacetophenone wie 3-Trifluormethylphenyl-4-cyanobenzylketon (auch als α-(4-Cyanophenyl)-3-trifluormethylacetophenon bezeichnet) ist ein wichtiger Ausgangsstoff zur Herstellung von Pflanzenschutzmitteln (siehe z. B. WO 00/18714).

Die JP 4168826 (JP-A-04270248) beschreibt ein Verfahren zur Herstellung von α-Phenylacetophenonen durch Kondensation von gegebenenfalls substituierten Benzoesäureestern mit substituierten Toluolen in Gegenwart wenigstens äquimolarer Mengen Base. Explizit beschrieben ist die Herstellung von 3-Trifluormethylphenyl-4-cyanobenzylketon durch Umsetzung eines Äquivalentes 3-Trifluormethylbenzoesäuremethylester mit einem Äquivalent 4-Tolunitril in N,N-Dimethylformamid. Als Base fungiert Natriumhydrid. Das beschriebene Verfahren ist vom sicherheitstechnischen Standpunkt aus problematisch, denn bei Reaktionen mit Natriumhydrid in N,N-Dimethylformamid kann es zu heftigen thermischen Zersetzungsreaktionen kommen, siehe Chemistry & Engineering 1982, 5, July 12 und 1982, 43, September 13. Außerdem ist Natriumhydrid an feuchter Luft selbstentzündlich und reagiert mit Feuchtigkeit sehr heftig zu Wasserstoff und Natronlauge. Daher dürfen die eingesetzten Edukte und Lösungsmittel nur einen extrem geringen Wassergehalt aufweisen. Die Verwendung anderer Basen wie Kalium-tert.-Butylat führt jedoch im Vergleich zu Natriumhydrid zu schlechteren Ausbeuten, wie man den Beispielen 3 und 3-2 entnehmen kann, welche die Herstellung von 3-Chlorphenyl-4-cyanobenzylketon beschreiben.

Eigene Untersuchungen der Anmelderin haben außerdem gezeigt, dass bei Verwendung anderer, in der JP 4168826 genannter Basen wie Kaliumcarbonat in N,N-Dimethylformamid keine Umsetzung und mit Natriumhydroxid in Dimethylsulfoxid ausschließlich Verseifung des Esters zur entsprechenden Säure als Nebenprodukt stattfindet.

Zur Vermeidung dieser Probleme schlägt die WO 00/18714 vor, derartige α-Phenylacetophenone durch Umsetzung von Acetophenonen mit aktivierten Halogenbenzolen umzusetzen. Nachteilig hieran sind die mäßigen Ausbeuten.

Der Erfindung lag daher die Aufgabe zugrunde, ein technisch sicheres, einfaches und wirtschaftliches Verfahren zur Herstellung von 3-Trifluormethylphenyl-4-cyanobenzylketon zu entwickeln, mit dem hohe Ausbeuten an Wertprodukt erreicht werden.

Es wurde nun überraschenderweise gefunden, dass sich 3-Trifluormethylphenyl-4-cyanobenzylketon in sehr guter Ausbeute unter technisch sicheren und milden Reaktionsbedingungen herstellen lässt, wenn man einen 3-Trifluormethylbenzoesäure-C₁-C₂-alkylester mit 4-Tolunitril in einem aprotischen polaren Lösungsmittel oder einem aprotisch polaren Lösungsmittelgemisch in Gegenwart wenigstens äquimolarer Mengen Kalium-C₁-C₄-alkoholaten eines primären C₁-C₄-Alkohols umsetzt.

Demnach betrifft die Erfindung ein Verfahren zur Herstellung von 3-Trifluormethylphenyl-4-cyanobenzylketon durch Umsetzung eines 3-Trifluormethylbenzoesäure-C₁-C₂-alkylesters mit 4-Tolunitril in einem aprotischen polaren Lösungsmittel oder einem aprotisch polaren Lösungsmittelgemisch in Gegenwart wenigstens einer äquimolaren Menge einer Base, **dadurch gekennzeichnet, dass** die Base ausgewählt ist unter Kaliumalkoholaten primärer C₁-C₄-Alkanole.

Zu den Kaliumalkoholaten primärer C₁-C₄-Alkohole zählen Kaliummethylat, Kaliumethylat, Kalium-n-propylat und Kalium-n-butylat. Bevorzugt ist Kaliummethylat.

In der Regel setzt man die Base wenigstens in äquimolarer Menge, bezogen auf 4-Tolunitril ein. Vorzugsweise setzt man 1,1 bis 5 Äquivalente Base, insbesondere 1,5 bis 4 Äquivalente und ganz besonders bevorzugt 2,01 bis 3 Äquivalente Base, bezogen auf 4-Tolunitril ein.

Bevorzugter 3-Trifluormethylbenzoesäure-C₁-C₂-alkylester ist 3-Trifluormethylbenzoesäuremethylester, der im Handel erhältlich ist. 4-Tolunitril ist ebenfalls im Handel erhältlich.

Das Reaktionsmedium ist erfindungsgemäß ein aprotisch, polares Lösungsmittelsystem, das auch Gemische verschiedener aprotisch, polarer Lösungsmittel und Gemische aprotisch, polarer Lösumgsmittel mit aprotisch unpolaren Lösungsmitteln umfasst. Der Anteil an unpolarem Lösungsmittel wird in der Regel 50 Vol.-%, insbesondere 20 Vol.-% nicht übersteigen. Der Anteil an aprotisch polaren Lösungsmittel in dem erfindungsgemäß einzusetzendem Lösungsmittel beträgt daher in der Regel wenigstens 50 Vol.-% und vorzugsweise wenigstens 80 Vol.-%. Zu den Beispielen für aprotische, polare Lösungsmittel zählen N,N-Dimethylamide aliphatischer C₁-C₄-Carbonsäuren wie N,N-Dimethylformamid oder N,N-Dimethylacetamid, N-Methyllactame wie N-Methylpyrrolidon, Dialkoxyalkane wie 1,2-Dimethoxyethan, Diethylenglykoldialkylether wie Diethylglykoldimethylether, Diethylglykoldiethylether, Sulfoxide wie Dimethylsulfoxid, Sulfolan oder Tetraalkylharnstoffe wie Tetramethylharnstoff. In einer bevorzugten Ausführungsform verwendet man wenigstens ein aprotisch polares Lösungsmittel als alleiniges Reaktionsmedium (> 99 Vol.-% bezogen auf die Gesamtlösungsmittel), das vorzugsweise ausgewählt ist unter 1,2-Dimethoxyethan, N,N-Dimethylformamid und Dimethylsulfoxid und hierunter besonders bevorzugt N,N-Dimethylformamid. Bevorzugte aprotisch unpolare Lösungsmittel sind beispielsweise aromatische Kohlenwasserstoffe wie Benzol, Toluol oder Xylole, cyclische Kohlenwasserstoffe wie Cyclohexan oder aliphatische Kohlenwasserstoffe wie n-Heptan, n-Hexan, Isohexan (kommerzielles Hexan-Isomerengemisch) Dekan, Petrolether, wobei aromatische Kohlenwasserstoffe, insbesondere Toluol und Xylole bevorzugt sind. In einer anderen bevorzugten Ausführungsform der vorliegenden Erfindung setzt man ein Lösungsmittelsystem ein, das neben dem aprotisch polaren Lösungsmittel insbesondere neben N,N-Dimethylformamid, 1 bis 50 Vol.-%, vorzugsweise 1 bis 20 Vol.-% und insbesondere 2 bis 15 Vol.-% wenigstens eines aprotisch unpolaren Lösungsmittels, insbesondere wenigstens eines aromatischen Kohlenwasserstoffs und speziell Toluol und/ oder Xylole umfasst. Dementsprechend ist der Anteil an aprotisch polaren Lösungsmitteln in dieser Mischung 50 bis 99 Vol.-% vorzugsweise 80 bis 99 Vol.-% und insbesondere 85 bis 98 Vol.-%. Der Zusatz von unpolaren Lösungsmitteln als Additiv zu den aprotisch polaren Lösungsmitteln erleichtert die technische Handhabbarkeit der Reaktion und führt insbesondere zu einer Verringerung der Viskosität der Reaktionsmischungen und unterdrückt zudem eine unerwünschte Belagsbildung an Kesselwänden und sonstigen Apparateteilen wie Rührer und Wärmeaustauscherflächen.

In der Regel setzt man die Ausgangsverbindungen 4-Tolunitril und 3-Trifluormethylbenzoesäure-C₁-C₂-alkylester in äquimolarem Verhältnis miteinander um, jedoch ist das Mengenverhältnis der Einsatzstoffe für den Erfolg der Reaktion von untergeordneter Bedeutung. Ein größerer Überschuss an 4-Tolunitril wird jedoch in der Regel vermieden, da er zur Bildung unerwünschter Nebenprodukte führen kann. In der Regel wird daher das Molverhältnis von 4-Tolunitril zu 3-Trifluormethylbenzoesäure-C₁-C₂-alkylester einen Wert von 2:1, insbesondere 1,5:1 nicht überschreiten. Umgekehrt kann man jedoch auch den Ester im Überschuss einsetzen, dies wird jedoch aus Kostengründen in der Regel vermieden. Das Molverhältnis von 4-Tolunitril zu Triflourmethylbenzoesäureester wird daher vorzugsweise ein Wert von 1:2 und insbesondere 1:1,5 nicht unterschreiten. In einer bevorzugten Ausführungsform beträgt das molare Verhältnis der Ausgangsverbindungen 4-Tolunitril und 3-Trifluormethylbenzoesäure-C₁-C₂-alkylester etwa 1:1, z. B. 1,1:1 bis 1:1,1.

Das erfindugsgemäße Verfahren erfolgt in der Regel bei Temperaturen unterhalb 100 °C vorzugsweise nicht oberhalb 60 °C, insbesondere im Bereich von +0 bis 40 °C.

Der Reaktionsdruck ist von untergeordneter Bedeutung. Häufig erfolgt die Umsetzung von 4-Tolunitril mit 3-Trifluormethylbenzoesäure-C₁-C₂-alkylester so, dass man zunächst das Lösungsmittel und die Base vorlegt und anschließend die Edukte, getrennt oder als Gemisch zugibt und gegebenenfalls erwärmt. Gibt man die Edukte nacheinander zu, so gibt man vorzugsweise zunächst das Nitril und anschließend den Ester zu. Die Reaktionsdauer hängt naturgemäß von der verwendeten Reaktionstemperatur, dem Reaktionsmedium und der Base ab und liegt in der Regel im Bereich von 0,5 bis 10 Stunden und insbesondere 0,5 bis 5 Stunden.

Die Umsetzung lässt sich in batch- oder semi-batch-Fahrweise durchführen.

Die Aufarbeitung des Reaktionsgemisches und die Abtrennung des Wertprodukts erfolgt nach den üblichen Techniken, beispielsweise durch Hydrolyse des bei der Reaktion entstehenden Kaliumenolats mit wässriger Säure wie Salzsäure, Schwefelsäure, Essigsäure, gefolgt von einer extraktiven Aufarbeitung. Die gegebenenfalls als Nebenprodukt gebildete 3-Trifluormethylbenzoesäure lässt sich durch alkalische Extraktion aus der organischen Phase entfernen. Die organische Phase mit dem Wertprodukt kann ohne weitere Aufarbeitung in nachfolgenden Umsetzungen eingesetzt werden. Gegebenenfalls kann man auch das Lösungsmittel entfernen und erhält die Zielverbindung in kristalliner Form.

Das erfindungsgemäße Verfahren weist gegenüber dem in der JP 4168826 beschriebenen Verfahren mehrere Vorteile auf. Zum einen kann auf die gefährliche Verwendung von Natriumhydrid in N,N-Dimethylformamid verzichtet werden. Ferner kann die organische Phase mit dem Wertprodukt unmittelbar nach der Abtrennung des gebildeten Nebenproduktes in Folgereaktionen eingesetzt werden, da das Reaktionsgemisch kein störendes Mineralöl aus dem Natriumhydrid enthält. Außerdem ist das neue Verfahren wirtschaftlicher, da bereits bei niedrigeren Reaktionstemperaturen höherer Ausbeuten an Wertprodukt erzielt werden. Bei Verwendung von Natrium-tert.-butylat oder Kalium-tert-butylat als Base sind hingegen höhere Reaktionstemperaturen erforderlich und man erhält zudem das Wertprodukt in schlechterer Ausbeute.

Die Erfindung wird durch die nachfolgenden Beispiele näher erläutert.

Herstellung von 3-Trifluormethylphenyl-4-cyanobenzylketon.

### Beispiel 1

In einem Reaktionsgefäß legte man bei 25 °C 10 Äquivalente N,N-Dimethylformamid vor und gab bei dieser Temperatur unter Rühren 2,5 Äquivalente Kaliummethylat zu. Anschließend gab man bei dieser Temperatur zunächst 1 Äquivalent 3-Trifluormethylbenzoesäuremethylester und danach 1 Äquivalent 4-Tolunitril zu. Man ließ unter den in Tabelle 1 angegebenen Bedingungen reagieren.

Sofern das Reaktionsgemisch auf Temperaturen oberhalb 25 °C erwärmt wurde, ließ man das Reaktionsgemisch zunächst auf Temperaturen unterhalb 40 °C abkühlen. Das Reaktionsgemisch wurde danach innerhalb 15 Minuten mit 3 Äquivalenten Salzsäure (10 Gew.-%) und dann mit 25 Äquivalenten Toluol versetzt. Nach Extraktion und Phasentrennung wurde der organische Extrakt mit 5 Gew.-%iger wässriger Natronlauge nachextrahiert. Die Ausbeute an Titelverbindung sind in Tabelle 1 angegeben.

| Temperatur [°C] | Reaktionszeit [h] | Ausbeute [%] |
|---|---|---|
| 50 | 1,5 | 86 |
| 25 | 4,0 | 82 |
| 25 | 18,0 | 83 |

### Vergleichsbeispiel 1

Man wiederholte Beispiel 1, verwendete aber anstelle von Kaliummethylat Natriummethylat. Die Reaktiontemperatur betrug 25 °C und die Reaktionszeit war 25 Stunden. Man erhielt die Titelverbindung in einer Ausbeute von 75 %.

### Vergleichsbeispiel 2

Man wiederholte Beispiel 1, verwendete aber anstelle von Kaliummethylat Natrium-tert.-butylat. Nach einer Reaktionszeit von 2 Stunden bei 80 °C erhielt man die Titelverbindung in 71 % Ausbeute.

### Vergleichsbeispiel 3

Man wiederholte Beispiel 1, verwendete aber anstelle von 2,5 Äquivalenten Kaliummethylat 2,1 Äquivalente festes Kalium-tert-butylat. Nach einer Reaktionszeit von 6,5 Stunden bei 50 °C unter Normaldruck erhielt man die Titelverbindung in 69,6 % Ausbeute.

## Patentansprüche

1. Verfahren zur Herstellung von 3-Trifluormethylphenyl-4-cyanobenzylketon durch Umsetzung eines 3-Trifluormethylbenzoesäure-C₁-C₂-alkylesters mit 4-Tolunitril in einem aprotischen polaren Lösungsmittel oder einem aprotisch polaren Lösungsmittelgemisch in Gegenwart wenigstens einer äquimolaren Menge einer Base, **dadurch gekennzeichnet, dass** die Base ausgewählt ist unter Kaliumalkoholaten eines primären C₁-C₄-Alkanols.

2. Verfahren nach Anspruch 1, **dadurch gekennzeichnet, dass** man 2,01 bis 3 Äquivalente Base, bezogen auf 4-Tolunitril verwendet.

3. Verfahren nach Anspruch 1 oder 2, **dadurch gekennzeichnet, dass** das aprotisch polaren Lösungsmittel ausgewählt ist unter Dimethylamiden aliphatischer C₁-C₄-Carbonsäuren, N-Methyllactamen, Tetramethylharnstoff, Dialkoxyalkanen, Diethylglykoldialkylethern und Dimethylsulfoxid.

4. Verfahren nach Anspruch 3, **dadurch gekennzeichnet, dass** das Lösungsmittel Dimethylformamid ist.

5. Verfahren nach einem der vorhergehenden Ansprüche, **dadurch gekennzeichnet, dass** das aprotische polare Lösungsmittelgemisch 1 bis 50 Vol.-% eines aromatischen Kohlenwasserstoffes umfasst.

6. Verfahren nach Anspruch 5, **dadurch gekennzeichnet, dass** das aprotisch polare Lösungsmittelgemisch als aprotisch polares Lösungsmittel Dimethylformamid und als aromatischer Kohlenwasserstoff Xylole und/oder Toluol umfasst.

7. Verfahren nach einem der vorhergehenden Ansprüche, **dadurch gekennzeichnet, dass** man die Umsetzung bei einer Temperatur im Bereich von 0 bis 40 °C durchführt.

## Claims

1. A process for preparing 3-trifluoromethylphenyl 4-cyanobenzyl ketone by reacting a C₁-C₂-alkyl 3-trifluoromethylbenzoate with 4-tolunitrile in an aprotic polar solvent or an aprotic polar solvent mixture in the presence of at least an equimolar amount of a base, wherein the base is selected from potassium alkoxides of a primary C₁-C₄-alkanol.

2. The process according to claim 1, wherein from 2.01 to 3 equivalents of base are used, based on 4-tolunitrile.

3. The process according to claim 1 or 2, wherein the aprotic polar solvent is selected from dimethylamides of aliphatic C₁-C₄-carboxylic acids, N-methyllactams, tetramethylurea, dialkoxyalkanes, diethyl glycol dialkyl ethers and dimethyl sulfoxide.

4. The process according to claim 3, wherein the solvent is dimethylformamide.

5. The process according to any of the preceding claims, wherein the aprotic polar solvent mixture comprises from 1 to 50% by volume of an aromatic hydrocarbon.

6. The process according to claim 5, wherein the aprotic polar solvent mixture comprises dimethylformamide as the aprotic polar solvent and xylenes and/or toluene as the aromatic hydrocarbon.

7. The process according to any of the preceding claims, wherein the reaction is carried out at a temperature in the range from 0 to 40°C.

## Revendications

1. Procédé pour la préparation de 3-trifluorométhylphényl-4-cyanobenzylcétone pour la transformation d'un ester C₁-C₂-alkylique due l'acide 3-trifluorométhylbenzoïque avec du 4-tolunitrile dans un solvant polaire aprotique ou un mélange de solvants polaires aprotiques en présence d'au moins une quantité équimolaire d'une base, **caractérisé en ce que** la base est choisie parmi les alcoolats de potassium d'un C₁-C₄-alcanol primaire.

2. Procédé selon la revendication 1, **caractérisé en ce qu'**on utilise 2,01 à 3 équivalents de base par rapport au 4-tolunitrile.

3. Procédé selon la revendication 1 ou 2, **caractérisé en ce que** le solvant polaire aprotique est choisi parmi les diméthylamides d'acides C₁-C₄-carboxyliques aliphatiques, les N-méthyllactames, la tétraméthylurée, les dialcoxyalcanes, les diéthylglycoldialkyléthers et le diméthylsulfoxyde.

4. Procédé selon la revendication 3, **caractérisé en ce que** le solvant est du diméthylformamide.

5. Procédé selon l'une quelconque des revendications précédentes, **caractérisé en ce que** le mélange de solvants polaires aprotiques comprend 1 à 50% en volume d'un hydrocarbure aromatique.

6. Procédé selon la revendication 5, **caractérisé en ce que** le mélange de solvants polaires aprotiques comprend comme solvant polaire aprotique du diméthylformamide et comme hydrocarbure aromatique des xylènes et/ou du toluène.

7. Procédé selon l'une quelconque des revendications précédentes, **caractérisé en ce qu'**on réalise la transformation à une température dans la plage de 0 à 40°C.
